# EUROPEAN PATENT APPLICATION

(11) **EP 1 625 848 A1**
(43) Date of publication of application: **15.02.2006**
(21) Application number: 05290804.3
(22) Date of filing: 12.04.2005
(51) Int. Cl.: A61K 31/515, A61P 9/10, A61P 25/00

(54) **Composition and method for enhanced delivery of 5,5-diphenyl barbituric acid**

(30) Priority: 10.08.2004 US 600327 P
(71) Applicant: Taro Pharmaceuticals North America, Inc., Grand Cayman (KY)
(72) Inventor: Levitt, Barrie, Mamaroneck New York 10543 (US); Moros, Daniel, Larchmont New York 10538 (US); Yacobi, Avraham, Englowood New Kersey 07631 (US); Gutman, Daniella, Rishon Lezion Israel 75241 (IL)
(74) Representative: Chajmowicz, Marion

(57) **Abstract**

The present invention relates to a composition and a method of delivering a barbituric acid derivative to the central nervous system of a mammal in need of treatment for neurological conditions. In particular, the present invention relates to a method of administering an oral dosage form of a sodium salt of 5,5-diphenyl barburtic acid to enhance the bioavailability of 5,5-diphenyl barbituric acid and brain delivery of same.

## Description

### Field of the Invention

The present invention relates to a composition and a method for providing improved bioavailability and enhanced delivery of a barbituric acid derivative to the central nervous system of a mammal. In particular, the present invention relates to a method of administering an oral dosage form comprising a salt form of 5,5-diphenyl barbituric acid selected from the group consisting of sodium, potassium and lithium to enhance the bioavailability of 5,5-diphenyl barbituric acid and brain delivery of same.

### Background of the Invention

5,5-diphenyl barbituric acid (DPB) is a barbituric acid derivative and was first prepared in 1935 *(*McElvain *et al*. "5,5-Diphenylbarbituric Acid" J. Am. Chem. Soc. 1935, 57:1301-04). The therapeutic use of DPB was initially overlooked because it lacks a purported hypnotic activity. DPB was later shown to possess an anticonvulsant activity (Merritt *et al.* "Experimental determination of anticonvulsive activity of chemical compounds" Epilepsia, 1945, 3, 751-75). To this end, Raines *et al.* ("The effects of 5,5-diphenyl barbituric acid on experimental seizures in rats: correlation between plasma and brain concentrations and anticonvulsant activity" Epilepsia, 1975, 16, 575-81) compared the intraperitoneal and oral administrations of DPB and correlated the plasma DPB concentrations with the anticonvulsant activity. It was found that oral gavage of DPB is considerably less potent when compared to intraperitoneal administration of DPB, which correlates well with the observation that oral absorption for DPB is poor. DPB is known to be 1/100 less soluble as compared to phenobarbital in aqueous solution at pH 7. *(See,* Epilepsia, 1973, 186, 315-22). The low oral absorption of DPB contributes to its sub-optimal bioavailability and so far has limited the use of DPB as an oral therapeutic agent.

Prodrugs of DPB include 1,3-dimethoxymethyl-5,5-diphenyl-barbituric acid (DMMDPB) and monomethoxymethyl-5,5-diphenylbarbituric acid (MMMDPB) which are metabolized to form DPB *in vivo*. Both DMMDPB and MMMDPB have been used as prodrugs to provide circulating levels of DPB. Other than the known intravenous and intraperitoneal administrations of DPB, there is no other dosage form of DPB that permits optimal and sustain levels of DPB.

There have been several reports regarding the potential use of several barbituric acid derivatives in the treatment of neurological conditions that are common in human. These neurological conditions include, *inter alia*, cerebral ischemia, head trauma, stroke, epilepsy and essential tremor. U.S. Pat. No. 4,628,056 describes the use of non-sedating barbituric acid derivatives to treat neurological conditions such as convulsions, seizures, nervous stress and anxiety. In light of its demonstration as a powerful anticonvulsant in mammals, and with a good correlation between the ability to prevent seizures in animals and in humans, DPB has potential use as a pharmacological agent for seizure disorders. Even at high therapeutic doses, this class of non-sedating barbituric acid derivatives is accompanied by remarkably little hypnotic and sedative effects. U.S. Pat. No. 6,756,379 describes the use of DMMDPB and its metabolites as neuroprotective agents against neurological conditions such as cerebral ischemia and head trauma. WO 2004/052350 describes the use of DMMDPB, MMMDPB, DPB, and derivatives of these compounds for the treatment of movement disorders; more specifically, essential tremor. In this study, reduction in essential tremor was found to correlate with plasma concentrations of MMMDPB and DPB. Accordingly, DPB is a potential pharmacological agent that may be useful in treating neurological conditions such as movement disorders, including essential tremor.

Studies have also suggested that the non-sedating barbituric acid derivatives (e.g., DPB) may prevent neuronal injury in ischemia by inhibiting the ischemia-induced uncontrolled release of neurotransmitters such as GABA. Earlier studies performed on isolated neural systems in aplysia and the hippocampus of the rat have shown that DPB can suppress neural repetitive firing in both systems at concentrations lacking significant effects on GABA neurotransmission. These effects may be attributable to enhancement of an outgoing membrane potassium current. Studies of DPB on cat motor nerve terminal function show a suppression of repetitive discharges similar to the effects of phenytoin, a compound which acts on sodium channels in the nerve membrane. It is generally speculated that improving the bioavailability and sustaining the blood levels of barbituric acid derivatives such as DPB may contribute to the beneficial effects of these drugs in the treatment of neurological conditions.

There is no prior art disclosing an oral dosage form of DPB that can sustain blood levels of DPB and thus exert its effects in the central nervous system. There is a continuing need to provide a composition and a method of enhancing the bioavailability of DPB and brain delivery of the same to the central nervous system in human.

### Summary of the Invention

It is an object of the present invention to provide an enhanced and efficient delivery of a barbituric acid derivative (i.e., DPB) to the central nervous system in a mammal by administrating a salt form of DPB in an oral dosage form. Preferably, the salt form of DPB is a salt selected from the group consisting of sodium, potassium and lithium. Preferably, the sodium salt of DPB is administered at a dose sufficient to ensure a sustained elevation of plasma concentration of DPB in human.

It is another object of the present invention to provide a method whereby a salt form of barbituric acid derivative (i.e., DPB) is utilized to deliver DPB to the central nervous system (i.e., bypassing the blood-brain barrier) and thus entering the central nervous system and cerebrospinal fluid.

It is another object of the present invention to provide a method for treating a neurological condition comprising the step of administering a sodium salt form of DPB so as to deliver DPB to the central nervous system.

It is yet another object of the present invention to provide a composition comprising a salt form of DPB that can be used to improve *in vivo* delivery of DPB. In particular, the present oral dosage form provides the advantages of improved bioavailability and increased brain delivery of DPB *in vivo*.

Accordingly, the present invention provides a method for treating a neurological condition in a mammal, comprising the step of administering an oral dosage form that comprises a salt form of 5,5-diphenyl barbituric acid to a mammal in a sufficient amount to provide an improved bioavailability for 5,5-diphenyl barbituric acid characterized by:
a) AUC₀₋₄₈ of 5,5-diphenyl barbituric acid that is at least about 2.5 times greater than that seen after the oral administration of the same amount of a free acid form of 5,5-diphenyl barbituric acid; or
b) Cₘₐₓ of 5,5-diphenyl barbituric acid that is at least about 1.5 times greater than that seen after the oral administration of the same amount of a free acid form of 5,5-diphenyl barbituric acid.

Preferably, the salt form of 5,5-diphenyl barbituric acid is sodium 5,5-diphenyl barbiturate, potassium 5,5-diphenyl barbiturate or lithium 5,5-diphenyl barbiturate. More preferably, the salt form of 5,5-diphenyl barbituric acid is sodium 5,5-diphenyl barbiturate.

Preferably, the AUC₀₋₄₈ of 5,5-diphenyl barbituric acid is at least about 3 times greater than that seen after the oral administration of the same amount of a free acid form of 5,5-diphenyl barbituric acid. More preferably, the AUC₀₋₄₈ of 5,5-diphenyl barbituric acid is at least about 4 times greater than that seen after the oral administration of the same amount of a free acid form of 5,5-diphenyl barbituric acid.

Preferably, the Cₘₐₓ of 5,5-diphenyl barbituric acid is at least about 2 times greater than that seen after the oral administration of the same amount of a free acid form of 5,5-diphenyl barbituric acid. More preferably, the Cₘₐₓ of 5,5-diphenyl barbituric acid is at least about 3 times greater than that seen after the oral administration of the same amount of a free acid form of 5,5-diphenyl barbituric acid.

Preferably, the salt form of 5,5-diphenyl barbituric acid is administered in a sufficient amount to provide a brain concentration of 5,5-diphenyl barbituric acid that is at least about 2.5 times greater than that seen after the oral administration of the same amount of a free acid form of 5,5-diphenyl barbituric acid. More preferably, the brain concentration of 5,5-diphenyl barbituric acid is at least about 3 times greater than that seen after the oral administration of the same amount of a free acid form of 5,5-diphenyl barbituric acid. More preferably, the brain concentration of 5,5-diphenyl barbituric acid is at least about 4 times greater than that seen after the oral administration of the same amount of a free acid form of 5,5-diphenyl barbituric acid.

Preferably, the salt form of 5,5-diphenyl barbiturate (e.g. the sodium form) is administered to provide a brain concentration of 5,5-diphenyl barbituric acid of at least about 20 µg/g at 24 hours after the administration. More preferably, the salt form of 5,5-diphenyl barbiturate (e.g. the sodium form) is administered to provide a brain concentration of 5,5-diphenyl barbituric acid of at least about 20 µg/g at 36 hours after the administration. More preferably, the salt form of 5,5-diphenyl barbiturate (e.g. the sodium form) is administered to provide a brain concentration of 5,5-diphenyl barbituric acid of at least about 8 µg/g at 48 hours after the administration.

Preferably, the oral dosage form is a tablet, pill, capsule, caplet, powder, granule or gel.

Preferably, the salt form of 5,5-diphenyl barbiturate (e.g. the sodium form) administered is at a dosage from about 5 mg/kg to about 200 mg/kg. More preferably, the salt of 5,5-diphenyl barbiturate (e.g. the sodium form) administered is at a dosage from about 5 mg/kg to about 25 mg/kg. More preferably, the salt form of 5,5-diphenyl barbiturate (e.g. the sodium form) administered is at a dosage of about 10 mg/kg.

Preferably, the salt form of 5,5-diphenyl barbiturate (e.g. the sodium form) is administered in the amount of from about 60 mg to about 1,200 mg per day. More preferably, the salt form of 5,5-diphenyl barbiturate (e.g. the sodium form) is administered in the amount of from 60 mg to about 600 mg per day. More preferably, the salt form of 5,5-diphenyl barbiturate (e.g. the sodium form) is administered in the amount of about 450 mg per day.

Preferably, the neurological condition is selected from the group consisting of convulsion, brain ischemia, traumatic brain injury, stroke, spinal cord injury, seizure, epilepsy, anxiety, nervous strain and movement disorders. Preferably, the movement disorder is essential tremor, dystonia or Parkinson's disease. Preferably, the movement disorder is selected from the group consisting of tremor, dystonia, chorea, athetosis, blepharospasm, hemiballysmus, myoclonus, torticollis, and writer's cramp. Preferably, the mammal is a human.

Accordingly, the present invention provides an oral dosage form comprising a salt form of 5,5-diphenyl barbituric acid and a pharmaceutically acceptable excipient, said salt form of 5,5-diphenyl barbituric acid is at least one salt selected from the group consisting of sodium 5,5-diphenyl barbiturate, potassium 5,5-diphenyl barbiturate and lithium 5,5-diphenyl barbiturate. Preferably, the salt form of 5,5-diphenyl barbituric acid is sodium 5,5-diphenyl barbiturate. More preferably, the oral dosage form is selected from the group consisting of a tablet, pill, capsule, caplet, powder, granule and gel.

Accordingly, the present invention provides a method for preparing a salt form of 5,5-diphenyl barbituric acid, comprising the steps of:
a) dissolving 5,5-diphenyl barbituric acid in an organic solvent selected from the group consisting of tetrahydrofuran, 2-methyltetrahydrofuran, diethyl ether, diisopropyl ether, tert-butyl methyl ether, dimethoxyethane, dioxane, diethylene glycol dimethyl ether, benzene, toluene, xylene, chlorobenzene, dichlorobenzene, ethanol, n-propyl alcohol, ethylene glycol, 1,3-butanediol, ethylene glycol monomethyl ether, N,N-dimethylformamide, N,N-dimethylacetamide, N-methylpyrrolidone, N,N-dimethylimidazoline, dimethylsulfoxide, sulfolane, acetonitrile and combinations thereof;
b) adding a base to the organic solvent, said base is at least one base selected from the group consisting of sodium hydroxide, potassium hydroxide and lithium hydroxide to the dissolved 5,5-diphenyl barbituric acid solution to form a salt form of 5,5-diphenyl barbituric acid; and
c) isolating the salt form of 5,5-diphenyl barbituric acid.

Preferably, the organic solvent is tetrahydrofuran. Preferably, the base and the dissolved 5,5-diphenyl barbituric acid in step b) are present at a molar ratio of about 1:1. Preferably, the isolating step is performed by filtration. Preferably, the present invention further comprises the step of washing the isolated salt form of 5,5-diphenyl barbituric acid. Preferably, the isolated salt form is sodium 5,5-diphenyl barbiturate.

### Brief Description of the Drawings

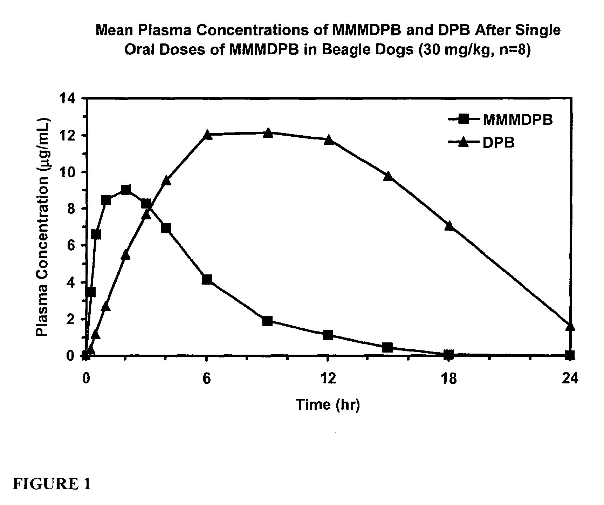

Figure 1 illustrates the mean plasma concentrations of MMMDPB and DPB after oral doses of MMMDPB (30 mg/kg) in Beagle dogs

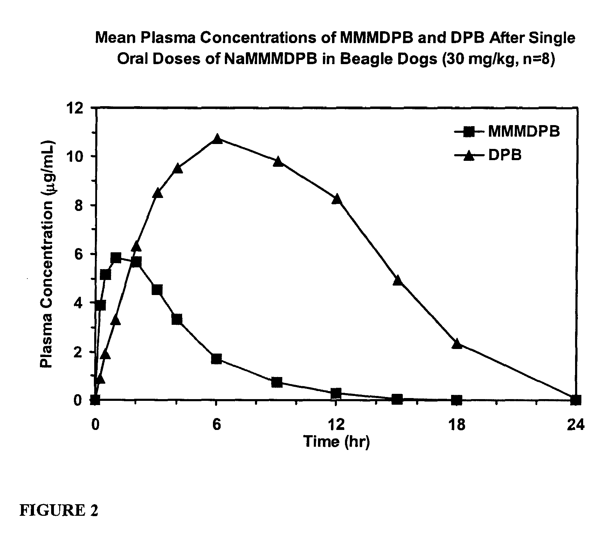

Figure 2 illustrates the mean plasma concentrations of MMMDPB and DPB after oral doses of NaMMMDPB (30 mg/kg) in Beagle dogs

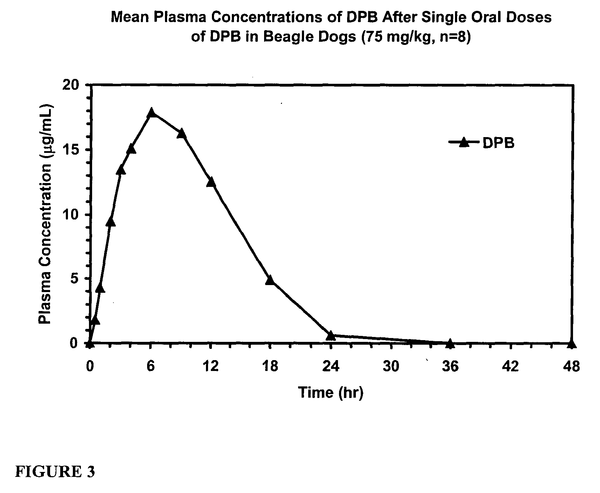

Figure 3 illustrates the mean plasma concentrations of DPB after oral doses of DPB (75 mg/kg) in Beagle dogs

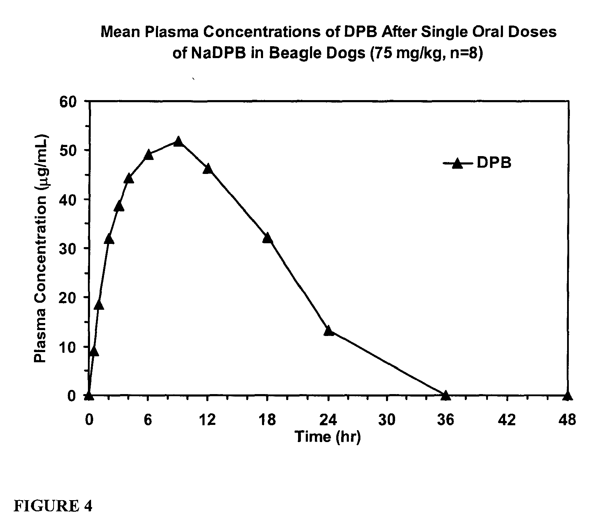

Figure 4 illustrates the mean plasma concentrations of DPB after oral doses of NaDPB (75 mg/kg) in Beagle dogs

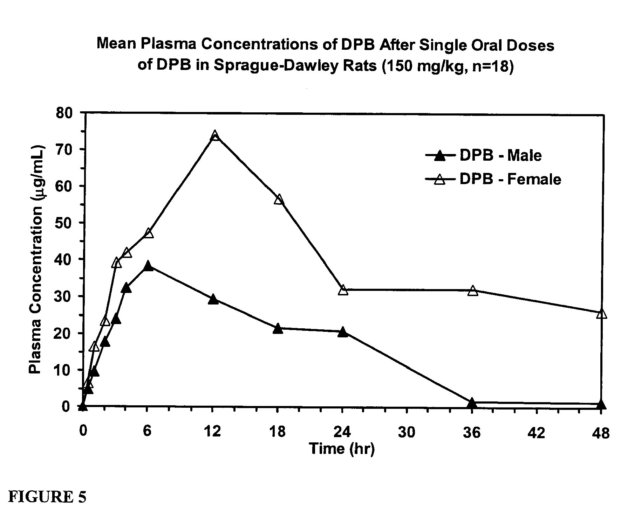

Figure 5 illustrates the mean plasma concentrations of DPB after oral doses of DPB (150 mg/kg) in Sprague-Dawley rats

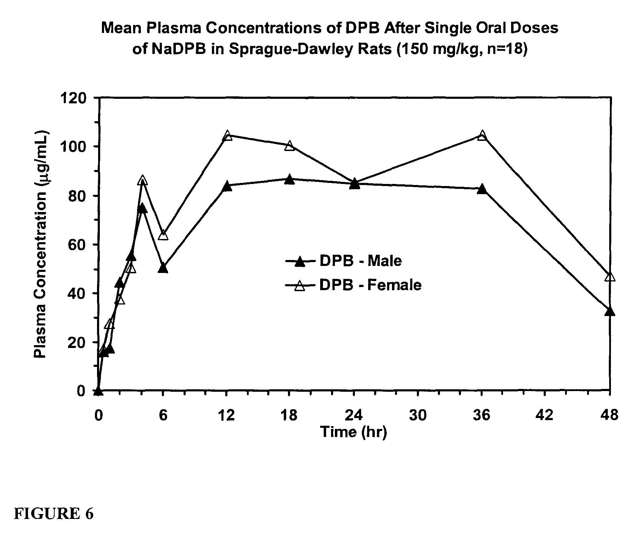

Figure 6 illustrates the mean plasma concentrations of DPB after oral doses of NaDPB (150 mg/kg) in Sprague-Dawley rats

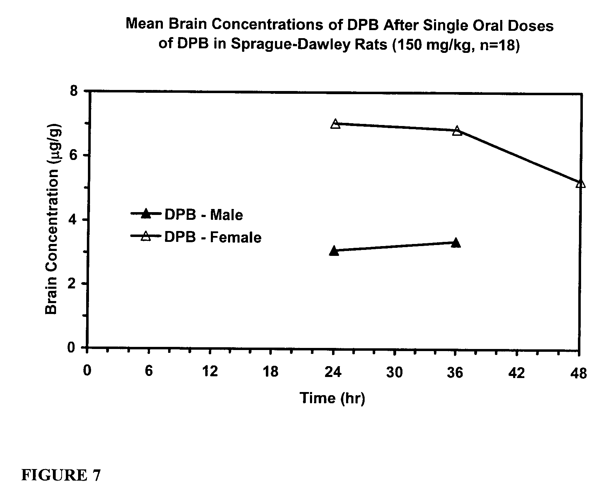

Figure 7 illustrates the mean brain concentration of DPB after oral doses of DPB (150 mg/kg) in Sprague-Dawley rats

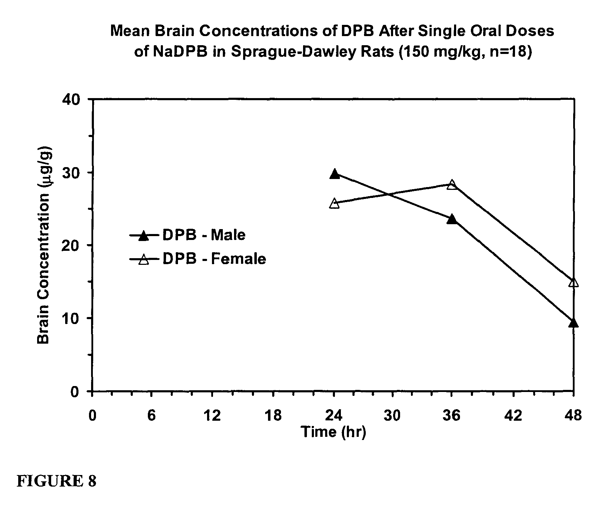

Figure 8 illustrates the mean brain concentration of DPB after oral doses of NaDPB (150 mg/kg) in Sprague-Dawley rats

The following detailed description, and the drawings to which it refers, are provided for the purpose of describing and illustrating certain examples or embodiments of the invention only and are not intended to exhaustively describe or show all possible embodiments or examples of the invention.

### Detailed Description of the Invention

**Definitions:** "DMMDPB" is 1,3-dimethoxymethyl-5,5-diphenyl barbituric acid; "MMMDPB" is monomethoxymethyl-5,5-diphenyl barbituric acid; "DPB" is 5,5-diphenyl barbituric acid; "AUC" refers to the mean area under the plasma concentration-time curve; "AUC₀₋ₜ" refers to area under the concentration-time curve from time zero to the time of the last sample collection; "AUC₀₋₂₄" refers to area under the concentration-time curve from time zero to 24 hours; "AUC₀₋₄₈" refers to area under the concentration-time curve from time zero to 48 hours; "Cₘₐₓ" refers to maximum observed plasma concentration; "Tₘₐₓ" (or "tₘₐₓ") refers to the time to achieve the Cₘₐₓ; "t_{1/2}" refers to the apparent half-life and is calculated as (ln 2/Kₑₗ), where Kₑₗ refers to the apparent first-order elimination rate constant; "NaDPB" and "sodium salt of DPB" are used herein interchangeably, and refer to sodium 5,5-diphenyl barbiturate; "NaMMMDPB" and sodium salt of MMMDPB" are used herein interchangeably and refer to sodium monomethoxymethyl-5,5-diphenyl barbiturate; unless otherwise indicated, "mean plasma concentration" and "plasma concentration" are used herein interchangeably; unless otherwise indicated, "mean brain concentration" and "brain concentration" are used herein interchangeably; "GABA" refers to gamma-aminobutyric acid, inhibitory neurotransmitter; "BQL" refers to below quantitation limit of assay; "HPLC" refers to high performance liquid chromatography; "pharmaceutically acceptable" refers to physiologically tolerable materials, which do not typically produce an allergic or other untoward reaction, such as gastric upset, dizziness and the like, when administered to a mammal; "mammal" refers to a class of higher vertebrates comprising man and all other animals that nourish their young with milk secreted by mammary glands and have the skin usually more or less covered with hair; and "treating" is intended to encompass relieving, alleviating or eliminating at least one symptom of a neurological condition in a mammal.

The present inventors have unexpectedly discovered a novel method for delivering DPB to a patient in need of treatment for a neurological condition, comprising the step of administering a salt form of DPB in an oral dosage form; preferably, the oral dosage form comprises sodium 5,5-diphenyl barbiturate. The present composition and method offer significant clinical advantages over the prior art in that it provides an improved bioavailability and enhanced delivery of DPB to the central nervous system.

### Bioavailability Studies

### Calculations of AUC and Cₘₐₓ

In accordance with the present invention, pharmacokinetic parameters were calculated using standard non-compartmental methods, as implemented in WinNonlin™ 4.0.1. The mean, standard deviation (SD) and percent coefficient (CV (%)) of variations were calculated for plasma concentrations of MMMDPB and DPB for each sampling time and for each treatment. The mean, SD and CV (%) were used to calculate the AUC₀₋ₜ (µg.h/mL), Cₘₐₓ (µg/ml) (the maximum observed concentration), Tₘₐₓ (hours) (the time to reach that peak concentration) and Kₑₗ (the elimination rate constant) for each animal and each analyte.

Areas under the concentration-time curves (AUC) were determined with respect for each animal that received oral administration of either NaDPB, NaMMMDPB or their free acid counterparts. AUC₀₋ₜ was calculated using the linear trapezoidal rule, which employs an approximate integration formula. The area of each trapezoid was calculated, and the sum of all the areas of all the trapezoids yielded an estimate of the true area under the curve. (*See*, Gibaldi *et al*. Pharmacokinetics. 2^{nd} Ed. Marcel Dekker, Inc., 1982; Yeh *et al*., A comparison of numerical integrating algorithms by trapezoidal, lagrange, and spline approximations. J. Pharmacokinet Biopharm. 6: 79 (1978), the disclosure of which is herein incorporated by reference.) Cₘₐₓ and Tₘₐₓ were then determined for each concentration vs. time profile. Elimination rate constant (Kₑₗ) was calculated using regression analyses on the natural log (In) of plasma concentration values (y) versus time (x).

### Pharmacokinetic Profile

As noted above, the present invention resides in the discovery that a salt form of DPB has an improved pharmacokinetic profile that can simultaneously accomplish two results. First, the oral dosage form containing a salt form of DPB maintains therapeutic levels of the DPB over a time period of over 48-hour dosing period. Second, the oral dosage form containing a salt form of DPB maintains optimal delivery of DPB to the central nervous system (e.g., brain) where neurological conditions occur.

In order to obtain these benefits, it is necessary to prepare a salt form of DPB as an oral dosage form of DPB to achieve certain pharmacokinetic parameters, when compared to the oral dosage form of the corresponding free acid form of DPB. The oral dosage form of the salt form of DPB, in particular the sodium 5,5-diphenyl barbiturate, significantly improves the bioavailability by increasing the pharmacokinetic profile (e.g., AUC₀₋ₜ and Cₘₐₓ) for DPB. In addition to increasing the peak DPB blood levels (i.e., Cₘₐₓ), it is important that the total amount of DPB absorbed (total drug absorption is referred to as AUC or area under the curve) be increased for the oral dosage form of the salt form of DPB as well.

AUC for the oral dosage form of a salt form of 5,5-diphenyl barbituric acid (such as sodium 5,5-diphenyl barbiturate) increases at least about 2.5 fold greater, when compared to that for the oral dosage form of the free acid form of 5,5-diphenyl barbituric acid. All of the AUCs over 24-hour, 36-hour and 48-hour intervals exhibit an increase for the oral dosage form of the salt form of 5,5-diphenyl barbituric acid. Preferably, the oral dosage form contains sufficient amount of the salt form of 5,5-diphenyl barbituric acid that provides AUC of at least about 3 fold greater, when compared to that for the oral dosage form of the free acid form of 5,5-diphenyl barbituric acid. More preferably, the oral dosage form contains sufficient amount of the salt form of 5,5-diphenyl barbituric acid that provides AUC of at least about 4 fold greater, when compared to that for the oral dosage form of the free acid form of 5,5-diphenyl barbituric acid. Thus, with respect to the extent of absorption, the oral dosage form of a salt form of 5,5-diphenyl barbituric acid of this invention should be considered improved bioavailability for DPB.

Cₘₐₓ for the oral dosage form of a salt form of 5,5-diphenyl barbituric acid (such as sodium 5,5-diphenyl barbiturate) increases at least about 1.5 fold greater, when compared to that for the oral dosage form of the free acid form of 5,5-diphenyl barbituric acid. All of the Cₘₐₓ over 24-hour, 36-hour and 48-hour intervals exhibit an increase for the oral dosage form of the salt form of 5,5-diphenyl barbituric acid. Preferably, the oral dosage form contains sufficient amount of the salt form of 5,5-diphenyl barbituric acid that provides Cₘₐₓ of DPB at least about 2.5 fold in male and at least about 1.5 fold in female greater, when compared to those for the oral dosage form of the free acid form of 5,5-diphenyl barbituric acid. More preferably, the oral dosage form contains sufficient amount of the salt form of 5,5-diphenyl barbituric acid that provides Cₘₐₓ of at least about 3 fold greater, when compared to that for the oral dosage form of the free acid form of 5,5-diphenyl barbituric acid. Thus, with respect to the peak plasma concentration, the oral dosage form of a salt form of 5,5-diphenyl barbituric acid of this invention should be considered improved bioavailability for DPB.

An improvement in oral bioavailability of DPB is believed to be critical in maintaining its therapeutic efficacy, which is reflected by optimal levels of AUC and Cₘₐₓ. Adequate delivery of DPB to central nervous system is believed to play a significant role in its effects on the central nervous system in neurological conditions in patients. It is contemplated that a plasma level of at least about 0.5 µg/ml is desirable. More preferably, it is contemplated that an optimal plasma level may be about 10 µg/ml to about 50 µg/ml of DPB.

It is noted that an oral dosage form comprising a sodium salt of MMMDPB does not increase bioavailability of DPB. The AUC₀₋ₜ and Cₘₐₓ of DPB were in fact lower after oral administration of sodium salt form of MMMDPB, as compared to the oral administration of the free acid form of MMMDPB (135.10 µg.hr/mL vs. 193.18 µg.hr/mL and 7.28 µg/mL vs. 9.39 µg/mL, respectively) (*See*, Table 1). Accordingly, the sodium salt form of MMMDPB fails to increase the bioavailability of DPB.

Preferably, the salt form of 5,5-diphenylbarbituric acid is administered in a sufficient amount to provide a brain concentration of 5,5-diphenylbarbituric acid that is at least about 2.5 times greater than that seen after the oral administration of the same amount of a free acid form of 5,5-diphenylbarbituric acid. More preferably, the brain concentration of 5,5-diphenylbarbituric acid is at least about 3 times greater than that seen after the oral administration of the same amount of a free acid form of 5,5-diphenylbarbituric acid. More preferably, the brain concentration of 5,5-diphenylbarbituric acid is at least about 4 times greater than that seen after the oral administration of the same amount of a free acid form of 5,5-diphenylbarbituric acid.

The present oral dosage form of a salt form of DPB provides an optimal delivery of DPB to central nervous system (e.g., brain). Preferably, administration of sodium 5,5-diphenyl barbiturate permits a brain concentration of at least about 20 µg/g of DPB 24 hours after the administration. Preferably, administration of sodium 5,5-diphenyl barbiturate permits a brain concentration of at least about 20 µg/g of DPB 36 hours after the administration. Preferably, administration of sodium 5,5-diphenyl barbiturate permits a brain concentration of at least about 8 µg/g of DPB 48 hours after the administration.

Without wishing to be bound by any theory, it is believed that the sodium salt of DPB is better absorbed through the intestinal and hence increases the plasma concentration in a faster manner as well as more in magnitude. It is further believed that a higher plasma concentration of DPB leads to a quicker drug entrance into the central nervous system. Carrier-mediated active membrane transport may explain the passage of some drugs across cell membranes. Transcellular fluxes are often formed by the active transport of Na⁺ across epithelial cells. When Na⁺ is actively transported, the drugs (such as DPB) may be enhanced to cross the epithelial cells in the intestinal system, ultimately lead to an increase in plasma concentration. If carrier-mediated transport is responsible for the improved bioavailability of DPB, the enhancement appears to be structural specific. A similar enhancement does not occur with MMMDPB; despite the fact that DPB and MMMDPB share an identical core structure, differing only by a methoxymethyl functional group.

In general, orally administered medicines are preferred for ease of use, cost efficiency and patient compliance. However, oral ingestion is often not compatible with many hydrophobic drugs. Moreover, this route of administration is not preferred when drugs absorbed from the gastrointestinal tract may be extensively metabolized by the liver before they gain access to the general circulation. We surprisingly found that a salt form of DPB (i.e., sodium 5,5-diphenyl barbiturate) is better in sustaining plasma concentrations of DPB so as to deliver optimal plasma concentration of DPB. The beneficial advantages of oral administration of sodium salt of DPB are parallel to that of intravenous administration. Infusion of drugs also has its disadvantages. Strict asepsis must be maintained to avoid infection, pain may accompany the injection, and it is often more expensive and less safe than oral medication.

In accordance with the present invention, the present invention provides a method of delivering a barbituric acid derivative (i.e., DPB) to the central nervous system including the brain. In mammals, brain keeps its environment constant by a blood-brain barrier. The blood-brain barrier separates the brain from the blood circulation and is involved in the homeostasis of the brain. The blood-brain barrier and the blood-cerebrospinal fluid barrier often preclude or slow the entrance of drugs into the central nervous system. The blood-brain barrier is composed of various cell types like endothelial cells, astrocytes, microglial cells, perivascular macrophages, and pericytes. The cerebral and endothelial cells form the morphological and functional basis of the blood-brain barrier. The bulk of the brain and the spinal cord is surrounded by a specially secreted clear fluid called the cerebrospinal fluid. Drug substances need to move across the blood-brain barrier before they can find their way into the cerebrospinal fluid where they are free to diffuse into the tissues of the brain. The entry of drugs such as barbituric acid derivatives into the central nervous system is restricted by the blood-brain barrier. We surprisingly found that oral administration of the sodium salt form of DPB enables the DPB to enter readily into the central nervous system as evidenced by the appearance in the brain. It is believed that the high delivery of DPB to the central nervous system provides a rapid and effective treatment for neurological conditions by this class of barbituric acid derivative.

The present invention is directed to a method of treating neurological conditions in a mammal. More particularly, the present invention provides a method of treating neurological damages in a patient by providing an improved bioavailability with an effective amount of DPB. According to the present invention, the term "patient" will encompass any mammal requiring treatment with DPB, particularly a human patient suffering from neurological disorder.

For the purpose of the present invention, neurological conditions include, but not limited to, convulsion, brain ischemia, traumatic brain injury, stroke, spinal cord injury, seizure, epilepsy, anxiety, nervous strain, and movement disorders. Movement disorders include a wide variety of disease states and physiological conditions. Non-limiting examples include various dyskinesias such as tremor, dystonia, chorea, athetosis, blepharospasm, as well as hemiballysmus, myoclonus, and focal dystonias, such as writer's cramp and torticollis. These excessive otherwise abnormal involuntary movements may vary significantly in rate, frequency, periodicity and progressionary character. Such movements may be seen in sometimes overlapping disorders such as Parkinson's disease; essential tremor, a.k.a. benign tremor or familial tremor; tic disorders, e.g., Tourette's syndrome; idiopathic dystonia (inducing writer's cramp), progressive supranuclear palsy and Wilson's disease. Essential tremor is one of the most common forms of tremor and of all movement disorders and it affects approximately 1-22% of elder population. Parkinson's disease is a progressive disorder with a prevalence of 1-2% in people over the age of 50. Preferably, the neurological condition is brain ischemia, epilepsy, essential tremor, or Parkinson's disease.

The dosage for the salt form of DPB or pharmaceutically acceptable salts thereof in the compositions of the invention will vary depending on several factors, including, but not limited to, the age, weight, and species of the patient, the general health of the patient, the severity of the symptoms in neurological injury, whether the composition is being administered alone or in combination with other agents, the incidence of side effects and the like.

While it is possible for the salt form of DPB to be administered alone, it is preferably present as a pharmaceutical composition. Preferably, the compositions of the present invention comprise at least one sodium salt of DPB, as defined above, together with one or more acceptable carriers thereof and optionally other therapeutic agents.

The above-mentioned method may be practiced by administration of the compound itself (e.g., salt form of DPB) in a combination with other active ingredients in a pharmaceutical composition. Other therapeutic agents suitable for use herein are any compatible drugs that are effective by the same or other mechanisms for the intended purpose, or drugs that are complementary to those of the present agents, e.g., other barbituric acid derivatives, particularly MMMDPB, DMMDPB or other non-sedative barbiturates. The compounds utilized in combination therapy may be administered simultaneously, in either separate or combined formulations, or at different times than the present compounds, e.g., sequentially, such that a combined effect is achieved. The amounts and regime of administration will be adjusted by the practitioner, by preferably initially lowering their standard doses and then titrating the results obtained. The therapeutic method of the invention may be used in conjunction with other therapies as determined by the practitioner.

### Pharmaceutical Composition Comprising Sodium, Potassium, and Lithium Salts of DPB

The pharmaceutical composition of the present invention comprises a salt form of 5,5-diphenyl-barbituric acid and a pharmaceutically acceptable excipient therefor. Preferably, the salt form is either sodium salt, potassium salt, lithium salt and the like. More preferably, the salt form is a sodium salt form of DPB.

The pharmaceutical composition of the present invention can be administered orally in the form of dry oral dosage forms. In accordance with the present invention, the salt form of DPB may be formulated into a variety of pharmaceutical compositions and dosage forms for therapeutic uses, especially in the treatment of a neurological condition. The pharmaceutical composition of the present invention can be administered orally in the form of tablets, pills, capsules, caplets, powders, granules, gels and the like. The oral dosage forms of the present pharmaceutical composition can be prepared by techniques known in the art and contains a therapeutically effective amount of a salt form of DPB.

Pharmaceutical compositions of the present invention may contain one or more pharmaceutical acceptable excipients. Excipients are added to the composition for a variety of purposes. The compositions may conveniently be presented in unit dosage form and may be prepared by any method known in the art. Such methods include the step of bringing the active ingredient into association with the carrier which itself may encompass one or more accessory ingredients. In general, the formulations are prepared by uniformly and intimately bringing into association the active ingredient with liquid carriers or finely divided solid carriers or both, and then if necessary shaping the product. Various unit dose and multidose containers, e.g., sealed ampules and vials, may be used, as is well known in the art *(See,* Remington's Pharmaceutical Sciences, 19th Edition, Alfonso R. Gennaro ed., Mack Publishing Company, Easton, Pa., Eighteenth edition (1995), the disclosure of which is incorporated by reference).

Preferably, the pharmaceutical composition is a dry oral dosage form. Preferably, the pharmaceutical composition is an oral dosage form selected from the group consisting of tablet, pill, capsule, caplet, powder, granule, and gel. Dry dosage forms may include pharmaceutically acceptable additives, such as excipients, carriers, diluents, stabilizers, plasticizers, binders, glidants, disintegrants, bulking agents, lubricants, plasticizers, colorants, film formers, flavouring agents, preservatives, dosing vehicles, and any combination of any of the foregoing.

Tablets and pills can additionally be prepared with enteric coatings and other release-controlling coatings so long as it provides a similar pharmacokinetic profile. The coating may be colored with a pharmaceutically accepted dye. The amount of dye and other excipients in the coating liquid may vary and will not impact on the tablets or pills. The coating liquid generally comprises film-forming polymers such as hydroxy-propyl cellulose, hydroxypropylmethyl cellulose, cellulose ester or ether, in acrylic polymer or a mixture of polymers. The coating solution is generally an aqueous solution that may further comprising propylene glycol, sorbitan monoleate, sorbic acid, fillers such as titanium dioxide, a pharmaceutically acceptable dye.

Diluents increase the bulk of a solid pharmaceutical composition and may make a pharmaceutical dosage form containing the composition easier for the patient and caregiver to handle. Diluents for solid compositions include, for example, microcrystalline cellulose (e.g. AVICEL® , microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit® ), potassium chloride, powdered cellulose, sodium chloride, sorbitol and talc.

Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. KLUCEL® ), hydroxypropyl methyl cellulose (e.g. METHOCEL® ), liquid glucose, magnesium aluminum silicate, maltodextrin, methylcellulose, polymethacrylates, povidone (e.g. KOLLIDON® , PLASDONE® ), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. AC-DI-SOL® , PRIMELLOSE® ), colloidal silicon dioxide, croscarmellose sodium, crospovidone (e.g. KOLLIDON® , POLYPLASDONE® ), guar gum, magnesium aluminum silicate, methyl cellulose, microcrystalline cellulose, polacrilin potassium, powdered cellulose, pregelatinized starch, sodium alginate, sodium starch glycolate (e.g. EXPLOTAB® ) and starch.

Glidants can be added to improve the flow properties of non-compacted solid compositions and improve the accuracy of dosing. Excipients that may function as glidants include colloidal silicon dioxide, magnesium trisilicate, powdered cellulose, starch, talc and tribasic calcium phosphate.

When a dosage form such as a tablet is made by compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease release of the product from the die. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl famarate, stearic acid, talc and zinc stearate. Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid ethyl maltol, and tartaric acid.

Compositions may also be colored using any pharmaceutically acceptable colorant to improve their appearance and/or facilitate patient identification of the product and unit dosage level.

Selection of excipients and the amounts to use may be readily determined by formulation scientists based upon experience and consideration of standard procedures and reference works in the field. The solid compositions of the present invention include powders, granulates, aggregates and compacted compositions. The preferred route of the present invention is oral. The dosages may be conveniently presented in unit dosage form and prepared by any of the methods well-known in the pharmaceutical arts. Dosage forms include solid dosage forms like tablets, pills, powders, caplets, granules, capsules, sachets, troches and lozenges. An especially preferred dosage form of the present invention is a tablet.

The present invention provides a method for preparing a pharmaceutical composition comprising the steps of: a) dissolving 5,5-diphenyl barbituric acid in an organic solvent selected from the group consisting of tetrahydrofuran, 2-methyltetrahydrofuran, diethyl ether, diisopropyl ether, tert-butyl methyl ether, dimethoxyethane, dioxane, diethylene glycol dimethyl ether, benzene, toluene, xylene, chlorobenzene, dichlorobenzene, ethanol, n-propyl alcohol, ethylene glycol, 1,3-butanediol, ethylene glycol monomethyl ether, N,N-dimethylformamide, N,N-dimethylacetamide, N-methylpyrrolidone, N,N-dimethylimidazoline, dimethylsulfoxide, sulfolane, acetonitrile and combination thereof; b) adding a basic solution to the organic solvent, said base is at least one base selected from the group consisting of sodium hydroxide solution, potassium hydroxide solution and lithium hydroxide solution to the dissolved 5,5-diphenyl barbituric acid solution to form a salt of 5,5-diphenyl barbituric acid; and c) isolating the salt of 5,5-diphenyl barbituric acid. Preferably, the salt is a sodium salt of 5,5-diphenyl barbituric acid.

The dissolution step of 5,5-diphenyl barbituric acid may be carried out in various solvents. Examples of solvents used include ethers such as tetrahydrofuran (THF), 2-methyl-tetrahydrofuran, dimethoxyethane, dioxane and diethylene glycol dimethyl ether; aromatic hydrocarbons such as benzene, toluene, xylene, chlorobenzene and dichlorobenzene; alcohols such as ethanol, n-propyl alcohol, ethylene glycol, 1,3-butanediol and ethylene glycol monomethyl ether; amides such as N,N-dimethylformamide, N,N-dimethylacetamide, N-methylpyrrolidone and N,N-dimethylimidazoline; hydrocarbons containing sulfur such as dimethylsulfoxide and sulfolane, and water; or polar aprotic solvents such as acetonitrile and the like. Preferably, the organic solvent is tetrahydrofuran.

In the above reaction, approximately a 1:1 molar ratio of a 5,5-diphenyl barbituric acid and a base is used. The reaction is carried out at a temperature that will be adjusted by a person skilled in the art to the base used and to the solvent used.

The isolating step may be performed by filtering. Other convenient methods may be adopted by one skilled in the art. Optionally, the precipitation may be enhanced by stirring and cooling. Preferably, the solution is cooled to about 4°C. Preferably, the solution is stirred for about 2 hours at room temperature followed by cooling to 4°C. Preferably, the present invention further comprises the step of washing the isolated salt of 5,5-diphenyl barbituric acid.

The precise amount of sodium salt of DPB administered to a patient may vary depending upon the degree of the disease and the size of the patient. A normal-weight (~ 60 kg) adult may be started a dosage from about 5 mg/kg to about 200 mg/kg. Preferably, the amount of the sodium salt of DPB is about 5 mg/kg to about 25 mg/kg. More preferably, the amount of the sodium salt of DPB is about 10 mg/kg. Tablets, capsules, lozenges and other dosage forms preferably contain unit dosage of sodium salt of DPB.

The daily dose of the pharmaceutical composition of this invention administered to a host in a single dose can be in the amounts from about 60 mg to about 1,200 mg. Preferably, the daily dose is about 60 mg to about 600 mg. More preferably, the daily dose is about 450 mg.

Having now generally described this invention, the same will be better understood by reference to the following Examples, which are provided herein solely for purposes of illustration only and are not intended to be limiting of the invention or any embodiment thereof, unless so specified.

### EXAMPLES

### Example 1 Preparation of Salt Forms of DPB

### Sodium salt

### Materials:

| Compound | Molecular weight | Weight (grams) | mMoles | Volumes |
|---|---|---|---|---|
| DPB | 280 | 46.35 | 166 | |
| NaOH | 40 | 6.60 | 165 | |
| THF | | | | 1,500 mL + 150 mL |
| DI water | | | | 25 mL |

| | | | | |
|---|---|---|---|---|
| THF: tetrahydrofuran; DI water: deionized water | | | | |

### Procedure:

DPB was dissolved in 1,500 mL THF. The turbid solution was filtered through folded filter paper. Sodium hydroxide solution was prepared by dissolving in a mixture of 150 mL THF and 25 mL water. The sodium hydroxide solution was added drop-wise to the DPB solution over a period of 0.5 hour. The sodium salt of DPB formed and precipitated from the solution. The mixture was stirred for about 2 hours at room temperature and then cooled to 4⁰C and stirred at that temperature for an additional 2 hours. The product was filtered and washed with cold THF. 42.57 grams wet product was obtained. The collected salt was dried in a vacuum oven at 50°C to constant weight of 40.12 grams. Yield: 80%.

### Potassium Salt

### Materials:

| Compound | Molecular weight | Weight (grams) | mMoles | Volumes |
|---|---|---|---|---|
| DPB | 280 | 9.18 | 32.8 | |
| KOH | 56.1 | 2.16 | 32.8 | |
| THF | | | | 300 mL + 30 mL |
| DI water | | | | 5 mL |

| | | | | |
|---|---|---|---|---|
| THF: tetrahydrofuran; DI water: deionized water | | | | |

### Procedure:

DPB was dissolved in 300 mL THF. The turbid solution was filtered through folded filter paper. Potassium hydroxide solution was prepared by dissolving in a mixture of 30 mL THF and 5 mL water. The potassium hydroxide solution was added drop-wise to the DPB solution over a period of 0.5 hour. The potassium salt of DPB formed and precipitated from the solution. The mixture was stirred for about 2 hours at room temperature and then cooled to 4°C and stirred at that temperature for an additional 2 hours. The product was filtered and washed with cold THF. 10.11 grams wet product was obtained. The collected salt was dried in a vacuum oven at 50°C to constant weight.
Yield: 96.9%.

### Lithium Salt

### Materials:

| Compound | Molecular weight | Weight (grams) | mMoles | Volumes |
|---|---|---|---|---|
| DPB | 280 | 0.972 | 3.31 | |
| LiOH.H₂O | 41.96 | 0.142 | 3.31 | |
| THF | | | | 30 mL + 3 mL |
| DI water | | | | 1 mL |

| | | | | |
|---|---|---|---|---|
| THF: tetrahydrofuran; DI water: deionized water | | | | |

DPB was dissolved in 30 mL THF. The turbid solution was filtered through folded filter paper. Lithium hydroxide solution was prepared by dissolving in a mixture of 3 mL THF and 1 mL water. The lithium hydroxide solution was added drop-wise to the DPB solution over a period of 0.5 hour. The lithium salt of DPB formed and precipitated from the solution. The mixture was stirred for about 2 hours at room temperature and then cooled to 4°C and stirred at that temperature for an additional 2 hours. The product was filtered and washed with cold THF. 0.92 grams wet product was obtained. The collected salt was dried in a vacuum oven at 50°C to constant weight.
Yield: 97.2%.

### Example 2 Preparation of Salt Form of MMMDPB

### Sodium salt

22 grams (68 mmol) of MMMDPB were suspended in 330 mL of t-butyl methyl ether and 10 mL of methanol. The suspension was heated to 60°C and stirred at such temperature for 30 minutes. Then, 13 mL of 30 % solution of sodium methoxide were added drop-wise. During the addition the suspension turned to a clear solution and after some time the product started to precipitate. After the addition of the base was completed, the reaction mixture was cooled to room temperature and stirred for further four hours. Very fine crystals of the sodium salt were obtained after filtration.

The product (NaMMMDPB) was dried at 60°C in a vacuum oven for three hours. 18.4 grams of dry sodium salt were obtained of which purity by HPLC was determined to be 98.6%. Yield: 78.4%.

### Example 3

### Sodium Salt of MMMDPB Fails to Increase Bioavailability of MMMDPB and DPB

This study compared the pharmacokinetics of MMMDPB and DPB after oral administrations of MMMDPB (30 mg/kg) and sodium salt of MMMDPB (30 mg/kg) in dogs. Oral suspensions of MMMDPB and sodium salt of MMMDPB were prepared by suspending either MMMDPB or sodium salt of MMMDPB in 2 % (w/v) aqueous carobxymethyl cellulose (CMC). Eight (8) Beagle dogs were administered the oral suspensions of MMMDPB or sodium salt of MMMDPB by a single oral gavage.

Blood samples were obtained pre-dose and 0.25, 0.5, 1, 2, 3, 4, 6, 9, 12, 15, 18, and 24 hours after dosing. Venous blood (~1 mL) were drawn and collected in lithium heparin (anticoagulant). Blood samples were spun in a refrigerated centrifuge and plasma were stored at -80°C prior to analysis. MMMDPB and DPB were quantified in plasma using HPLC with Tandem Mass Spectrometry detection (LLOQ=0.25 µg/mL). Calibration curves were obtained using a weighted (1/C²) least squares linear regression analysis of peak area ratio (analyte/internal standard) versus the nominal concentration of the calibration standards. Concentrations were obtained by interpolation from the run defined calibration curve. Regressions and figures were generated by PE Sciex Analyst Version 1.2 software.

Plasma samples were thawed and subjected to solid phase extraction with 2 mL acetonitrile prior to HPLC method with Tandem Mass Spectrometry Detection. Chromatographic conditions were performed as followed:

| | |
|---|---|
| Chromatographic mode: | Reversed phase |
| Isocratic/gradient mode: | Isocratic |
| Mobile phase flow rate: | 1 mL/min |
| Back-pressure: | 140 bars (approximately) |
| Autosampler rising vol.: | 1,000 µl |
| Column: | Zorbax SBC 18 |
| Retention time: | 2.15 minutes (DPB) |
| | 3.46 minutes (MMMDPB) |

The AUC₀₋ₜ and Cₘₐₓ for MMMDPB were lesser after oral administration of sodium salt of MMMDPB, than after oral administration of MMMDPB (29.44 µg.hr/mL vs. 57.41 µg.hr/mL and 6.45 µg/mL vs. 10.22 µg/mL) *(See,* Table 1).

The AUC₀₋ₜ and Cₘₐₓ for DPB were similarly lesser after oral administration of sodium salt of MMMDPB, than after oral administration of MMMDPB (135.10 µg.hr/mL vs. 193.18 µg.hr/mL and 7.28 µg/mL vs. 9.39 µg/mL) (*See*, Table 1).

**Table 1: Mean Pharmacokinetic Parameters of MMMDPB AND DPB after Oral Administration of NaMMMDPB in Beagle Dogs (30 mg/kg, n=8)**

| Drug | Route | Dose | Analyte | Cₘₐₓ (µg/mL) | tₘₐₓ (hr) | AUC₀₋ₜ (µg·hr/mL) | t_{1/2} (hr) |
|---|---|---|---|---|---|---|---|
| MMMDPB | Oral | 30 mg/kg | MMMDPB | 10.22 | 3.25 | 57.41 | 2.59 |
| MMMDPB | Oral | 30 mg/kg | DPB | 13.91 | 9.39 | 193.18 | 4.59 |
| NaMMMDPB | Oral | 30 mg/kg | MMMDPB | 6.45 | 1.04 | 29.44 | 2.71 |
| NaMMMDPB | Oral | 30 mg/kg | DPB | 11.57 | 7.28 | 135.10 | 3.15 |

| | | | | | | | |
|---|---|---|---|---|---|---|---|
| AUC₀₋ₜ refers to AUC₀₋₂₄ in this study | | | | | | | |

Thus, these data show that oral administration of sodium salt of MMMDPB fails to increase the bioavailability of DPB.

### Example 4

### Sodium Salt of DPB Increases Bioavailability of DPB

We next examined the delivery of DPB to the vascular compartment by administrating directly DPB and compared it with the sodium salt of DPB. This study compared the pharmacokinetics of DPB after oral administration of DPB (75 mg/kg) or sodium salt of DPB (75 mg/kg) in Beagle dogs. Twenty-eight Beagle dogs were administered DPB and sodium salt of DPB by a single oral gavage (n=24). Blood samples were obtained pre-dose and 0.5, 1, 2, 3, 4, 6, 12, 18, 24, 36 and 48 hours after dosing. DPB was quantified in plasma using HPLC (LLOQ=0.25 µg/mL).

The AUC₀₋ₜ for DPB was greater (~4 fold) after oral administration of sodium salt of DPB, than after oral administration of DPB (882.8 µg.hr/mL vs. 222.1 µg.hr/mL) (*See*, Table 2).

The Cₘₐₓ for DPB was greater (~3 fold) after oral administration of sodium salt of DPB, than after oral administration of DPB (54.2 µg/mL vs. 18.7 µg/mL) *(See,* Table 2).

**Table 2: Mean Pharmacokinetic Parameters of DPB after Oral Administration of DPB and NaDPB in Beagle Dogs (75 mg/kg, n=28)**

| Drug | Route | Dose | Analyte | Cₘₐₓ (µg/mL) | tₘₐₓ (hr) | AUC₀₋ₜ (µg·hr/mL) | t_{1/2} (hr) |
|---|---|---|---|---|---|---|---|
| DPB | Oral | 75 mg/kg | DPB | 18.7 | 6.5 | 222.1 | 4.0 |
| NaDPB | Oral | 75 mg/kg | DPB | 54.2 | 7.8 | 882.8 | 5.9 |

| | | | | | | | |
|---|---|---|---|---|---|---|---|
| AUC₀₋ₜ refers to AUC₀₋₄₈ in this study | | | | | | | |

These data show that administration of sodium salt of DPB increases the bioavailability of DPB.

### Example 5

### Administration of DPB and Sodium Salt Form of DPB

We continued to examine the delivery of DPB to the vascular compartment by administrating directly DPB and compared it with the sodium salt of DPB. This study compared the pharmacokinetics of DPB after oral administration of DPB (150 mg/kg) and sodium salt of DPB (150 ng/kg) in rats. Fifty-four rats were administered DPB and sodium salt of DPB by a single oral gavage (n=54). Blood samples were obtained (approximately 1 mL each) were collected following each treatment from animal via the jugular vein. An indwelling catheter was used for blood sampling. The time-points were pre-dose, 0.15, 0.5, 1, 2, 3, 4, 6, 9, 12, 15, 18, 24, 36 and 48 hours after dosing. Each blood sample was collected into a tube containing lithium heparin and the samples were spun in a refrigerated centrifuge. The resultant plasma was stored at -80°C prior to analysis. DPB was quantified in plasma using HPLC (LLOQ=0.25 µg/mL).

Table 3 summarizes the pharmacokinetic parameters of DPB. As shown in Table 3, the AUC₀₋ₜ for DPB was greater in male (~4.5 fold) and female (~2 fold) after oral administration of sodium salt of DPB, than after oral administration of DPB (3414.7 µg.hr/mL vs. 775.5 µg.hr/mL and 4037.6 µg.hr/mL vs. 1945.9 µg.hr/mL) *(See,* Table3).

The Cₘₐₓ for DPB was greater in male (~2.5 fold) and female (~1.5 fold) after oral administration of sodium salt of DPB, than after oral administration of DPB (86.9 µg/mL vs. 38.3 µg/mL and 104.5 µg/mL vs. 74.0 µg/mL) *(See,* Table 3).

**Table 3: Mean Pharmacokinetic Parameters of DPB after Oral Administration of DPB and sodium salt of DPB in Sprague-Dawley Rats (150 mg/kg, n=72)**

| Drug | Route | Dose (mg/kg) | Analyte | Sex | Cₘₐₓ (µg/mL) | tₘₐₓ (hr) | AUC₀₋ₜ (µg·hr/mL) | t_{1/2} (hr) |
|---|---|---|---|---|---|---|---|---|
| DPB | Oral | 150 | DPB | Male | 38.3 | 6 | 775.5 | 6.6 |
| | | | | Female | 74.0 | 12 | 1945.9 | 33.2 |
| NaDPB | Oral | 150 | DPB | Male | 86.9 | 18 | 3414.7 | 17.6 |
| | | | | Female | 104.5 | 12 | 4037.6 | 32.9 |

| | | | | | | | | |
|---|---|---|---|---|---|---|---|---|
| AUC₀₋ₜ refers to AUC₀₋₄₈ in this study | | | | | | | | |

These data indicate that administration of sodium salt of DPB greatly increases the bioavailability of DPB.

### Example 6

### Sodium Salt of DPB Increases Brain Delivery of DPB

In this study, we measured the brain concentration of DPB. Brains were collected as soon as possible after euthanasia from each rat. They were stored in a freezer at -80°C prior to determination of DPB brain tissue concentrations. Table 4 summarizes the brain concentrations of DPB after oral administration of DPB and sodium salt of DPB in Sprague-Dawley rats.

Brain tissue mean concentrations of DPB are generally greater in female animals than in male animals following administration of NaDPB. As shown in Table 4, the brain concentration of DPB was greater at all the time intervals (i.e., 24, 36 and 48 hours) in male after oral administration of sodium salt of DPB, than after oral administration of DPB (29.91 µg/g vs, 3.09 µg/g; 23.64 µg/g vs, 6.84 µg/g; 9.46 µg/g vs, BQL, respectively) (*See*, Table 4).

The brain concentration of DPB was also greater at all the time intervals (i.e., 24, 36 and 48 hours) in female after oral administration of sodium salt of DPB, than after oral administration of DPB (25.80 µg/g vs, 7.04 µg/g; 28.40 µg/g vs, 6.84 µg/g; 14.94 µg/g vs, 5.24, respectively) *(See,* Table 4).

**Table 4: Mean Brain Concentrations (µg/g) of DPB after Oral Administration of DPB and Sodium Salt of DPB in Sprague-Dawley Rats (150 mg/kg, n=72)**

| Drug | Route | Dose | Analyte | Sex | Time (hr) | | | |
|---|---|---|---|---|---|---|---|---|
| | | | | | 18 | 24 | 36 | 48 |
| DPB | Oral | 150 mg/kg | DPB | Male | - | 3.09 | 3.36 | BQL |
| | | | | Female | - | 7.04 | 6.84 | 5.24 |
| NaDPB | Oral | 150 mg/kg | DPB | Male | - | 29.91 | 23.64 | 9.46 |
| | | | | Female | - | 25.80 | 28.40 | 14.94 |

| | | | | | | | | |
|---|---|---|---|---|---|---|---|---|
| BQL: Below quantification limit | | | | | | | | |

These data indicate that administration of sodium salt of DPB is rapidly absorbed through the digestive system, and increase the brain bioavailability of DPB at least many folds compared to that seen after oral administration of the free acid form of DPB.

Those skilled in the art will recognize, or be able to ascertain using no more than routine experimentation, many equivalents of the specific embodiments of the invention described herein. The disclosures of the cited publications are incorporated herein in their entireties by reference. It is to be understood, however, that the scope of the present invention is not to be limited to the specific embodiments described above. The invention may be practiced other than as particularly described and still be within the scope of the accompanying claims.

## Claims

1. An oral dosage form comprising a salt form of 5,5-diphenyl barbituric acid and a pharmaceutically acceptable excipient, wherein said salt form of 5,5-diphenyl barbituric acid is at lease one salt selected from the group consisting of sodium 5,5-diphenyl barbiturate, potassium 5,5-diphenyl barbiturate and lithium 5,5-diphenyl barbiturate.

2. The oral dosage form of claim 1, wherein the salt form of 5,5-diphenyl barbituric acid is sodium 5,5-diphenyl barbiturate.

3. The oral dosage form of claims 1 or 2, wherein the oral dosage form is selected from the group consisting of a tablet, pill, capsule, caplet, powder, granule, and gel.

4. Use of the oral dosage form of any of claims 1 to 3, for the manufacture of a medicament for treating a neurological condition in a mammal.

5. The use of claim 4, wherein the salt form of 5,5-diphenyl barbiturate administered is at a dosage from about 5 mg/kg to about 200 mg/kg.

6. The use of claim 4, wherein the salt form of 5,5-diphenyl barbiturate administered is at a dosage from about 5 mg/kg to about 25 mg/kg.

7. The use of claim 4, wherein the salt form of 5,5-diphenyl barbiturate administered is at a dosage of about 10 mg/kg.

8. The use of claim 4, wherein the salt form of 5,5-diphenyl barbiturate is administered in the amount of from about 60 mg to about 1,200 mg per day.

9. The use of claim 4, wherein the salt form of 5,5-diphenyl barbiturate is administered in the amount of from about 60 mg to about 600 mg per day.

10. The use of claim 4, wherein the salt form of 5,5-diphenyl barbiturate is administered in the amount of about 450 mg per day.

11. The use of claim 4, wherein the neurological condition is selected from the group consisting of convulsion, brain ischemia, traumatic brain injury, stroke, spinal cord injury, seizure, epilepsy, anxiety, nervous strain and movement disorders.

12. The use of claim 11, wherein the movement disorder is essential tremor, dystonia or Parkinson's disease.

13. The use of claim 11, wherein the movement disorder is selected from the group consisting of tremor, dystonia, chorea, athetosis, blepharospasm, hemiballysmus, myoclonus, torticollis, and writer's cramp.

14. The use of any of claims 4 to 13, wherein the mammal is a human.
